# EUROPÄISCHE PATENTANMELDUNG

(11) **EP 2 106 786 A1**
(43) Veröffentlichungstag der Anmeldung: **07.10.2009**
(21) Anmeldenummer: 08006866.1
(22) Anmeldetag: 04.04.2008
(51) Int. Cl.: A61K 9/08, A61K 31/05, A61K 31/715

(54) **Pharmazeutische Zubereitung mit permethyliertem Cyclodextrin**

(71) Anmelder: Roewer, Norbert, Univ.-Prof. Dr. med., 97082 Würzburg (DE); Broscheit, Jens, Dr. med., 97209 Würzburg (DE)
(72) Erfinder: Roewer, Norbert, Univ.-Prof. Dr. med., 97082 Würzburg (DE); Broscheit, Jens, Dr. med., 97209 Würzburg (DE)
(74) Vertreter: Glawe, Delfs, Moll

(57) **Zusammenfassung**

Gegenstand der Erfindung ist eine pharmazeutische Zubereitung zur Applikation eines pharmazeutischen Wirkstoffs. Erfindungsgemäß ist vorgesehen, dass die Zubereitung ein methyliertes Cyclodextrin mit einem Substitutionsgrad von wenigstens 2,5 Methylgruppen pro Glucopyranoseeinheit aufweist.

## Beschreibung

Die Erfindung betrifft eine pharmazeutische Zubereitung, wie beispielsweise Zubereitungen für die präoperative Anxiolyse oder für anästhetische Zwecke.

In der Pharmazie ist es ein häufiges Problem, einen pharmazeutischen Wirkstoff so zu formulieren, dass er mittels einer bestimmten Applikationsart in der gewünschten Konzentration und möglichst effizient an den vorgesehenen Wirkort gebracht wird. So muss ein für intravenöse Applikation vorgesehener Wirkstoff in gewissem Umfang wasserlöslich sein, damit überhaupt eine systemische Konzentration im Blut erreicht werden kann. Andererseits muss er in der Regel eine gewisse Lipophilie aufweisen, um am vorgesehenen Wirkort ggf. Zellmembranen durchdringen zu können. Ein lediglich gut wasserlöslicher Wirkstoff kann beispielsweise nach intravenöser Applikation eine hohe systemische Konzentration im Blut aufbauen, er wird aber beispielsweise bei zu geringer Lipophilie eine geringe Bioverfügbarkeit aufweisen, da am vorgesehenen Wirkort möglicherweise ein unzureichender Durchtritt durch die Zellmembran erfolgt.

Bei bestimmten Applikationsarten wie beispielsweise der transmucosalen Applikation kann die Bioverfügbarkeit eines Wirkstoffes darunter leiden, dass der Durchtritt durch die Mucosa zu langsam oder verzögert erfolgt und deswegen eine hinreichende systemische Konzentration des Wirkstoffes nicht oder zu langsam erreicht wird. Diese Problematik sei nachfolgend erläutert am Beispiel der sogenannten präoperativen Anxiolyse.

Vor einer Anästhesie und Operation eines Patienten wird regelmäßig eine Prämedikation durchgeführt. Deren vorrangiges Ziel ist die Stressreduktion bzw. -vermeidung durch eine sogenannte Anxiolyse. Die präoperative psychische Verfassung eines Patienten hat einschlägigen Untersuchungen zufolge erheblichen Einfluss auf das intraoperative Kreislaufverhalten und den postoperativen Schmerzmittelbedarf. Ungenügende Anxiolyse kann z.B. zu erhöhten Magensäuresekretion mit der Gefahr der Aspiration bei der Narkoseeinleitung führen. Dies kann lebensbedrohlich sein.

Zur Anxiolyse werden im Stand der Technik regelmäßig Benzodiazepine verwendet.

Die Prämedikation am Operationstag erfolgt in aller Regel peroral durch Einnahme eines schnell anflutenden Benzodiazepins, insbesondere Midazolam, etwa 45 bis 60 min vor A-nästhesiebeginn.

Die Bioverfügbarkeit einer peroralen Anwendung ist sehr variabel und damit schwer kalkulierbar. Fehldosierungen mit gegebenenfalls unzureichender Anxiolyse sind nicht auszuschließen.

Ferner ist die bei peroraler Gabe nötige Anflutungszeit zur Erzielung eines ausreichenden Plasmaspiegels (45 bis 60 min) nicht mehr vereinbar mit den Anforderungen an das Zeitmanagement in Krankenhäusern. Zur Erzielung eines hohen Auslastungsgrades der Operationseinrichtungen erfolgt der Abruf eines Patienten zur Operation regelmäßig mit einem Vorlauf von lediglich 15 min. Dieser geringe Vorlauf erlaubt keine hinreichende Anxiolyse durch perorale Benzodiazepin Gabe.

Es ist bereits vorgeschlagen worden (WO-A-01/30391), "Midazolam mit β-Cyclodextrin zu einer transmucosal anwendbaren Lösung zu formulieren.

Der Erfindung liegt die Aufgabe zu Grunde, eine pharmazeutische Zubereitung der eingangs genannten Art zu schaffen, die eine sichere und zuverlässige Applikation eines Wirkstoffes bei hinreichend zuverlässig vorhersagbaren Wirkprofil ermöglicht.

Erfindungsgemäß ist vorgesehen, dass die pharmazeutische Zubereitung einen pharmazeutischen Wirkstoff und ein methyliertes Cyclodextrin mit einem Substitutionsgrad von wenigstens 2,5 Methylgruppen pro Glucopyranoseeinheit aufweist.

Im Stand der Technik (WO A 01/30391) sind bereits Cyclodextrine als Hilfsstoffe vorgeschlagen worden, die pharmazeutische Wirkstoffe komplexieren, gegebenenfalls in wässriger Lösung stabilisieren bzw. löslich machen und die Membrangängigkeit erhöhen sollen.

Die vorliegende Erfindung hat erkannt, dass überraschender Weise spezielle Cyclodextrine, nämlich methylierte Cyclodextrine mit einem Substitutionsgrad von wenigstens 2,5 Methylgruppen pro Glucopyranoseeinheit, einerseits eine sehr gute Stabilisierung und Komplexierung auch lipophiler Wirkstoffe in wässriger Lösung ermöglichen und andererseits den Durchtritt der Wirkstoffe durch Membranen erleichtern.

Eine mögliche Erklärung für diese überraschend verbesserte Wirkung der hochgradig methylierten Cyclodextrine könnte sein, dass höher methylierte Cyclodextrine einen erhöhten lipophilen Charakter aufweisen und daher die Wechselwirkung mit der Membran und damit die Resorption des vom Cyclodextrin komplexierten Wirkstoffes erleichtern.

Die hohe Lipophilie der erfindungsgemäß verwendeten Cyclodextrine drückt sich in einem hohen logP-Wert aus, der den Verteilungskoeffizienten der entsprechenden Substanz in dem Lösemittelsystem Octanol/Wasser beschreibt. Dieser logP-Wert liegt erfindungsgemäß vorzugsweise wenigstens bei 5, weiter vorzugsweise wenigstens bei 7. Für das ein permethyliertes β-Cyclodextrin liegt dieser logP-Wert bei 9.

Cyclodextrine weisen sechs (α-Cyclodextrin), sieben (β-Cyclodextrin) oder acht (y-Cyclodextrin) Glucopyranoseeinheiten auf. Jede Glucopyranoseeinheit weist drei OH-Gruppen auf, die substituiert, im vorliegenden Fall methyliert werden können. Ein Substitutionsgrad von wenigstens 2,5 Methylgruppen pro Glucopyranoseeinheit bedeutet, das statistisch gesehen 2,5 dieser drei OH-Gruppen methyliert worden sind. Weitere bevorzugte Mindestsubstitutionsgrade sind 2,6 sowie 2,7, ferner 2,8 und 2,9. Permethyliertes Cyclodextrin ist vollständig substituiert, das heißt der Substitutionsgrad beträgt 3.

Unter den Cyclodextrinen ist β-Cyclodextrin besonders bevorzugt.

Die erfindungsgemäß verwendeten Cyclodextrine haben eine polare Außenfläche und eine hydrophobe Kavität (Hohlraum) im Inneren. Sie komplexieren den bevorzugt lipophilen pharmazeutischen Wirkstoff und machen ihn auf diese Weise in wässriger Phase löslich. Am pharmakologischen Wirkort setzt der Komplex den Wirkstoff so frei (möglicherweise durch ein "Andocken" des Cyclodextrins an die Membran), so dass die Membran ohne weiteres durchdrungen werden kann.

Die Aufnahme von Arzneimitteln in Zielzellen erfolgt häufig passiv, da hierfür normalerweise keine Transportsysteme über die Zellmembran zur Verfügung stehen. Der Grad des passiven Diffusionsflusses eines Arzneimittels durch eine biologische Membran wird wesentlich durch die Lipophilie des Arzneimittels sowie dessen Konzentrationsgradient an der Membran bestimmt. Diese beiden Bedingungen sind allerdings häufig gegenläufig. Hohe Lipophilie (und damit eine gute Fähigkeit zur passiven Durchdringung der Zellmembran am Wirkort) bedingt häufig eine geringe Wasserlöslichkeit, so dass kein großer Konzentrationsgradient über der als Barriere wirkenden Membran erzeugt werden kann, da das entsprechende Arzneimittel in der wässrigen Phase außerhalb der Zellmembran wenig löslich ist.

Bei der erfindungsgemäßen pharmazeutischen Zubereitung ist der bevorzugt lipophile pharmazeutische Wirkstoff durch das Cyclodextrin so komplexiert, dass er als Komplex gut wasserlöslich ist und damit in höherer Konzentration an die Membran gebracht werden kann. Einmal an die Membran herantransportiert, wird er offensichtlich in einer Art und Weise freigesetzt, dass die lipophilen Eigenschaften beim Durchdringen der Membran dominieren. Bei der erfindungsgemäßen Zubereitung erlaubt also der Komplex aufgrund seiner Wasserlöslichkeit die Einstellung eines hohen Konzentrationsgefälles über die Membranbarriere, gleichzeitig kann die Lipophilie des pharmazeutischen Wirkstoffes eine hohe Durchdringungsgeschwindigkeit der Membranbarriere bewirken. Die beiden im Stand der Technik in der Regel gegenläufigen Faktoren für eine gute Durchdringung einer Zellmembran am pharmakologischen Wirkort können erfindungsgemäß also in überraschender Weise synergistisch kombiniert werden.

Bevorzugt ist die Komplexbildungskonstante K des Komplexes aus pharmazeutischem Wirkstoff und Cyclodextrin zwischen 10 und 70 1/mol, weiter bevorzugt 15 und 65 l/mol, weiter bevorzugt zwischen 20 und 55 l/mol, besonders bevorzugt zwischen 30 und 40 l/mol. Komplexbildungskonstanten in diesem Bereich stellen eine hinreichende Stabilität des Komplexes in wässrigen Systemen sicher, erlauben aber die Freisetzung des pharmazeutischen Wirkstoffs an der Membran des vorgesehenen Wirkortes.

Der pH-Wert der erfindungsgemäßen pharmazeutischen Zubereitung liegt vorzugsweise zwischen 4 und 7, besonders bevorzugt zwischen 5,5 und 6,5. Dies entspricht dem pH-Wert der Schleimhäute, so dass eine beispielsweise eine transmucosale Applikation ohne Irritationen oder sonstige unangenehme Nebenwirkungen möglich ist.

Die erfindungsgemäße pharmazeutische Zubereitung kann verschiedene pharmazeutische Wirkstoffe umfassen. Bevorzugt ist es, wenn der pharmazeutische Wirkstoff einen logP-Wert von wenigstens 3 aufweist. Dies bedeutet, dass der dekadische Logarithmus des Verteilungsgleichgewichtes dieses Wirkstoffes in Octanol/Wasser-Mischungen wenigstens 3 beträgt. Es handelt sich somit um ein Maß für die Lipophilie des pharmazeutischen Wirkstoffes. Die logP-Werte erfindungsgemäß einsetzbarer pharmazeutischer Wirkstoffe können sehr viel höher sein, beispielhaft genannt seien lipophile Stoffe wie bspw. Halothan mit einem logP-Wert von etwa 200.

β-Cyclodextrin besitzt einen zur Aufnahme eines pharmazeutischen Wirkstoffs geeigneten Hohlraum mit einem Durchmesser von etwa 0,6 bis 0,65 nm und einer Höhe von etwa 0,79 nm. Bevorzugt sind somit Wirkstoffe, die sich an diese Hohlraumgröße anpassen können.

Wie oben beschrieben eignet sich eine erfindungsgemäße Zubereitung somit dafür, die Bioverfügbarkeit eines pharmazeutischen Wirkstoffes am vorgesehenen Wirkort zu erhöhen. Der Komplex der Zubereitung gelangt bei dieser Wirkweise (beispielsweise durch die Blutbahn) an den pharmazeutischen Wirkort, dort wird der Wirkstoff aus dem Komplex freigesetzt und durchdringt die Zellmembran. Das Cyclodextrin selbst kann die Zellmembran nicht durchdringen.

Gemäß einem weiteren Aspekt der Erfindung kann die erfindungsgemäße Zubereitung nicht nur verwendet werden, um die Bioverfügbarkeit durch Transport des Wirkstoffes (mittels des Cyclodextrinkomplexes) durch die Blutbahn an den Wirkort sicherzustellen, sondern um bei bestimmten Applikationsarten überhaupt erst die Einstellung einer hinreichend großen und im zeitlichen Profil hinreichend sicher vorhersagbaren systemischen Wirkstoffkonzentration zu ermöglichen. Bei dieser Variante der Erfindung setzt der Komplex der erfindungsgemäßen Zubereitung den Wirkstoff nicht am eigentlichen Wirkort frei, sondern verbessert beispielsweise bei einer transmucosalen Applikation den Durchtritt des Wirkstoffes durch die Mucosamembran, so dass schnell und vorhersagbar eine bestimmte systemische Wirkstoffkonzentration aufgebaut wird. Bei dieser Variante der Erfindung erfolgt somit die Freisetzung des Wirkstoffes aus dem Cyclodextrinkomplex bereits beim Durchtritt durch die Mucosamembran.

Die erfindungsgemäße Zubereitung eignet sich somit auch für eine Formulierung zur transmucosalen Applikation.

Eine pharmazeutische Zubereitung zur transmucosalen Applikation umfasst einen mit entsprechenden Hilfsstoffen formulierten pharmazeutischen Wirkstoff (gegebenenfalls auch mehrere Wirkstoffe) der bzw. die ganz oder zu wesentlichen Teilen durch Schleimhäute (insbesondere Nasen- und/oder Mundschleimhäute) systemisch aufgenommen wird bzw. werden. Die transmucosale Applikation hat den grundsätzlichen Vorteil, dass sie eine schnellere, zuverlässigere und besser kalkulierbare systemische Anflutung eines Wirkstoffes ermöglicht, da die Einstellung eines entsprechenden Serumspiegels nicht von den Besonderheiten und Unwägbarkeiten des Magen-Darm-Traktes abhängt. (First-Pass Effekt bedingt durch die Leberpassage nach enteraler Resorption). Die erfindungsgemäße Zubereitung kann insbesondere für eine transnasale, intralinguale, intestinale oder orovestibulare Applikation formuliert sein. Die intestinale Resorption kann durch diese Zubereitung auch profitieren.

Der pharmazeutische Wirkstoff ist besonders bevorzugt ein Benzodiazepin. Die Benzodiazepine können beispielsweise ausgewählt sein aus der Gruppe bestehend aus Alprazolam, Brotizolam, Chlordiazepoxid, Clobazam, Clonazepam, Clorazepam, Demoxazepam, Flumazenil, Flurazepam, Halazepam, Midazolam, Nordazepam, Medazepam, Diazepam, Nitrazepam, Oxazepam, Midazepam, Lorazepam, Prazepam, Quazepam, Triazolam, Temazepam und Loprazolam. Unter diesen Benzodiazepinen ist Midazolam besonders bevorzugt.

Der Anteil des Benzodiazepins an der pharmazeutischen Zubereitung kann bei einer bevorzugten Ausführungsform 0,5 bis 2 Gew.-%, insbesondere 1 Gew.-% betragen. Die Zubereitung ist bevorzugt eine wässrige Lösung.

Gegenstand der Erfindung ist ferner die Verwendung eines methylierten Cyclodextrins mit einem Substitutionsgrad von wenigstens 2,5 Methylgruppen pro Glucopyranoseeinheit zur Herstellung einer pharmazeutischen Zubereitung, beispielsweise zur transmucosalen Applikation. Bevorzugt ist ferner die erfindungsgemäße Verwendung zur Herstellung eines Medikaments zur präoperativen Anxiolyse.

Gemäß einem weiteren Aspekt der Erfindung kann eine erfindungsgemäße pharmazeutische Zubereitung verwendet werden, um einen nicht oder kaum wasserlöslichen Wirkstoff einer intravenösen Administration zugänglich zu machen. Dies sei am Beispiel von Propofol erläutert.

Propofol ist ein erstmals 1977 in der klinischen Praxis getestetes intravenöses Anästhetikum. Die Lösung des Anästhetikums in einer Fettemulsion (Handelsname Diprivan®) konnte den häufig beobachteten Venenschmerz bei der i.v. Applikation vermindern, so dass Propofol 1989 in die klinische Praxis eingeführt wurde.

Diese Propofol-Lipidemulsion enthält Sojaöl, Glycerol und Eiphosphatide. Venenschmerz bei der Injektion ist ein nach wie vor häufig auftretendes Problem. Zudem kann es zu schwerwiegenden allergischen Reaktionen kommen. Da die Formulierung als Fettemulsion mikrobielles Wachstum begünstigt, kann es bei einer Kontamination der Emulsion bereits nach kurzen Lagerungszeiten zu einer Sepsis nach Applikation kommen .

Erfindungsgemäß kann ein pharmazeutischer Wirkstoff als Komplex aus Cyclodextrin mit dem oben definierten Mindestmethylierungsgrad und Propofol gebildet werden, der all die genannten Nachteile nicht aufweist.

Wie im experimentellen Teil dargelegt wird, weist eine erfindungsgemäße Propofolformulierung erheblich niedrige equipotente Dosen im Vergleich zu der Lipidemulsion des Standes der Technik auf. Da keine Hilfsstoffe zur Formulierung einer Lipidemulsion benötigt werden, treten die beschriebenen Probleme einer möglichen Sepsis durch mikrobiellen Befall ebenfalls nicht auf.

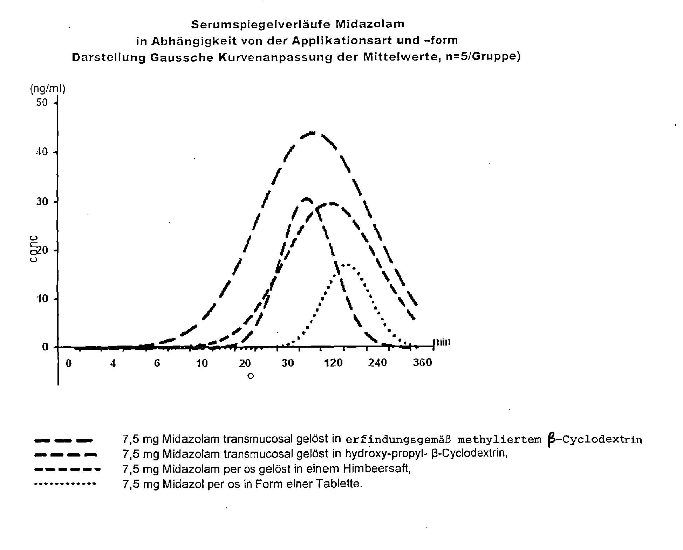

Ausführungsbeispiele der Erfindung werden nachfolgend beschrieben. Die Zeichnung zeigt den Serumspiegelverlauf von Midazolam für die beschriebene Ausführungsform der Erfindung und Vergleichsbeispiele.

### Beispiel 1

Midazolam-Zubereitung für transmucosale Applikation

Eine 1%ige Midazolam-Zubereitung für transnasale Applikation wird wie folgt formuliert:

| | |
|---|---|
| Midazolam: | 10 mg |
| Permethyliertes β-Cyclodextrin | |
| (Methylierungsgrad:3) | 150 mg |
| Hypromellose 400: | 1 mg |
| H₃PO₄, konz.: | 2,6 mg |
| NaOH 10%: | q.s. Zur Herstellung |
| | eines ph-Wertes von 4,2 |
| Kaliumsorbat: | 1,4 mg |
| Wasser: | ad 1 ml. |

Hypromellose 400 ist eine Hydroxypropylmethyl-Cellulose mit einem Molekulargewicht von etwa 400. Sie dient als Benetzungsmittel für die Schleimhäute. Der pH-Wert der so hergestellten Lösung beträgt 4,2.

Im vorliegenden Beispiel beträgt das Massenverhältnis zwischen dem Cyclodextrin und dem pharmazeutischen Wirkstoff 15:1. Im Rahmen der Erfindung sind allgemein Verhältnisse zwischen Cyclodextrin und pharmazeutischen Wirkstoff von 50:1 bis 10:1, weiter 40:1 bis 12:1, weiter 20:1 bis 12:1 bevorzugt.

Jeweils 0,75 ml dieser Lösung, (enthaltend 7,5 mg Midazolam) wurden einer Gruppe von fünf Probanden transmucosal appliziert.

Zum Vergleich mit dem Stand der Technik wurde drei weiteren Gruppen von jeweils fünf Probanden die gleiche Wirkstoffmenge von 7,5 mg Midazolam in folgender Weise appliziert:
**Vergleichsbeispiel 1:** Das permethylierte-β-Cyclodextrin des erfindungsgemäßen Beispiels wurde ersetzt durch Hydroxypropyl-β-Cyclodextrin.
**Vergleichsbeispiel 2**: 7,5 mg Midazolam per os gelöst in einem Himbeersaft.
**Vergleichsbeispiel 3**: 7,5 mg Midazolam per os in Form einer Tablette.

Bei dem Vergleichsbeispiel 1 wurde mit Ausnahme des Austauschs der Cyclodextrine die gleiche Rezeptur verwendet wie im Beispiel 1.

Die Mittelwerte der Serumspiegelverläufe von Midazolam als Funktion der Zeit nach der entsprechenden Applikation sind in der Figur dargestellt. Man erkennt, dass die erfindungsgemäße Zubereitung die schnellste Anflutung kombiniert mit der Erreichung des höchsten Serumspiegels und der längsten Wirkdauer.

Eingangs ist beschrieben worden, dass in Krankenhäusern häufig der Abruf von Patienten zu einer Operation mit einem Vorlauf von lediglich 15 min. erfolgt. Somit steht lediglich dieses Zeitfenster für eine anxiolytische Prämedikation zur Verfügung, da man ein schnell wirkendes Benzodiazepin wie Midazolam nicht einfach auf Verdacht geben kann, ohne zu wissen, wann die Operation tatsächlich stattfinden wird.

Die Zeichnung zeigt, dass lediglich die erfindungsgemäße Formulierung bereits nach 15 min. einen nennenswerten Serumspiegel aufgebaut hat, der bereits eine anxiolytische Wirkung hat. Sämtliche Applikationsformen des Standes der Technik (einschließlich der transmucosalen Applikation mit Hydroxypropyl-β-Cyclodextrin als Komplexbildner) fängt nach 15 min. überhaupt erst an, einen Serumspiegel aufzubauen. In der Praxis bedeutet dies, dass eine anxiolytische Prämetikation des Standes der Technik nach 15 min. praktisch noch nicht wirkt und damit ihren Zweck vollständig verfehlt.

### Beispiel 2

Propofol-Zubereitung für i.v. Applikation

Es werden zwei 1%ige Propofol Formulierungen zur Verfügung gestellt. Als Formulierung des Standes der Technik wurde eine unter dem Handelsnamen Diprivan® erhältliche Lipidemulsion zur Verfügung gestellt, die 1% Propofol in einer Emulsionsgrundlage aus Sojaöl, Glycerin und Eiphosphatid enthält.

Erfindungsgemäße Formulierung:

| | |
|---|---|
| Propofol: | 10 mg |
| Permethyliertes β-Cyclodextrin : | 80,2 mg |
| H₂PO₄, konz. : | 2,6 mg |
| NaOH 10%: | q.s. zur Einstellung |
| | eines pH-Wertes von 6,8 |
| Wasser: | ad 1 ml |

Diese Formulierung wird nachfolgend als CD-Propofol bezeichnet.

Je 12 Göttinger Mini-Schweine mit einem Körpergewicht zwischen 48 und 53 kg wurden randomisiert mit Diprivan® und CD-Propofol anästhesiert. Die Administration erfolgte mittels eines peripheren Venenkatheters.

Die Überwachung der Narkosetiefe erfolgte durch Cerebral State Monitoring (CSM) und entsprechende Aufzeichnung des Cerebral State Index (CSI). Als erwünschte Narkosetiefe wurde ein CSI von 40 bis 60 eingestellt. Die Überwachung der Narkose erfolgte ferner durch Aufnahme eines Elektrokardiogramms (ECG), Pulsoxymetrie (Ermittlung des SaO₂-Wertes und Capnometrie (Ermittlung des pCO₂-Wertes.

Die equipotenten Dosen zur Erzielung der o. g. Narkosetiefe betrugen 87,5 ± 12,5 mg bei der erfindungsgemäßen Formulierung gegenüber 225 ± 12,5 mg bei der Formulierung des Standes der Technik (Diprivan).

Die erfindungsgemäße Formulierung zeigt eine wesentlich schnellere An- und Abflutung. Die Wirkung der erfindungsgemäßen Formulierung setzt 1 min nach der Administration ein, bei der Formulierung des Standes der Technik beträgt dieser Zeitraum 2 min. Das Wirkungsende tritt bei der erfindungsgemäßen Formulierung nach 19 min ein, bei der Formulierung des Standes der Technik nach 25,5 min. Die erfindungsgemäße Formulierung bewirkt im Gegensatz zum Stand der Technik keinerlei Emulyse.

## Patentansprüche

1. Pharmazeutische Zubereitung zur Applikation eines pharmazeutischen Wirkstoffs, **dadurch gekennzeichnet, dass** sie einen pharmazeutischen Wirkstoff und ein methyliertes Cyclodextrin mit einem Substitutionsgrad von wenigstens 2,5 Methylgruppen pro Glucopyranoseeinheit aufweist.

2. Pharmazeutische Zubereitung nach Anspruch 1, **dadurch gekennzeichnet, dass** das Cyclodextrin ein β-Cyclodextrin ist.

3. Pharmazeutische Zubereitung nach Anspruch 1 oder 2, **dadurch gekennzeichnet, dass** das Cyclodextrin permethyliert ist.

4. Pharmazeutische Zubereitung nach einem der Ansprüche 1 bis 3, **dadurch gekennzeichnet, dass** sie einen pH-Wert von 4 bis 7, vorzugsweise 5,5 bis 6,5 aufweist.

5. Pharmazeutische Zubereitung nach einem der Ansprüche 1 bis 4, **dadurch gekennzeichnet, dass** die Komplexbildungskonstante K des Komplexes aus pharmazeutischem Wirkstoff und Cyclodextrin zwischen 10 und 70 l/M, vorzugsweise 15 und 65 l/M, weiter vorzugsweise 20 und 55 l/M, weiter vorzugsweise 30 und 40 l/M liegt.

6. Pharmazeutische Zubereitung nach einem der Ansprüche 1 bis 5, **dadurch gekennzeichnet, dass** der pharmazeutische Wirkstoff einen logP-Wert von wenigstens 3 aufweist.

7. Pharmazeutische Zubereitung nach einem der Ansprüche 1 bis 6, **dadurch gekennzeichnet, dass** sie für eine transmucosale Applikation formuliert ist.

8. Pharmazeutische Zubereitung nach Anspruch 6, **dadurch gekennzeichnet, dass** sie für eine transnasale, intralinguale, intestinale oder orovestibulare Applikation formuliert ist.

9. Pharmazeutische Zubereitung nach einem der Ansprüche 1 bis 7, **dadurch gekennzeichnet, dass** der pharmazeutische Wirkstoff ein Benzodiazepin umfasst.

10. Pharmazeutische Zubereitung nach Anspruch 8, **dadurch gekennzeichnet, dass** das Benzodiazepin ausgewählt ist aus der Gruppe bestehend aus Alprazolam, Brotizolam, Chlordiazepoxid, Clobazam, Clonazepam, Clorazepam, Demoxazepam, Flumazenil, Flurazepam, Halazepam, Midazolam, Nordazepam, Medazepam, Diazepam, Nitrazepam, Oxazepam, Midazepam, Lorazepam, Prazepam, Quazepam, Triazolam, Temazepam und Loprazolam.

11. Pharmazeutische Zubereitung nach Anspruch 8 oder 9, **dadurch gekennzeichnet, dass** der Gehalt des Benzodiazepins 0,5 bis 2 Gew.-% beträgt.

12. Pharmazeutische Zubereitung nach einem der Ansprüche 1 bis 7, **dadurch gekennzeichnet, dass** sie eine Formulierung eines Anästhetikums für i.v.-Applikation ist.

13. Pharmazeutische Zubereitung nach Anspruch 12, **dadurch gekennzeichnet, dass** sie Propofol enthält.

14. Methyliertes Cyclodextrin mit einem Substitutionsgrad von wenigstens 2,5 Methylgruppen pro Glucopyranoseeinheit zur Herstellung einer pharmazeutischen Zubereitung.

15. Methyliertes Cyclodextrin nach Anspruch 14 zur Herstellung eines Medikaments zur präoperativen Anxiolyse oder eines Anästhetikums für i.v. Applikation.
